# EUROPÄISCHE PATENTANMELDUNG

(11) **EP 1 153 569 A2**
(43) Veröffentlichungstag der Anmeldung: **14.11.2001**
(21) Anmeldenummer: 01111590.4
(22) Anmeldetag: 12.05.2001
(51) Int. Cl.: A61B 3/00

(54) **Operationsmikroskop**

(30) Priorität: 08.05.2000 CH 9772000
(71) Anmelder: Leica Microsystems AG, 9435 Heerbrugg (CH)
(72) Erfinder: Sander, Ulrich, Dr., 9445 Rebstein (CH)
(74) Vertreter: Reichert, Werner F., Dr.

(57) **Zusammenfassung**

Die Erfindung betrifft ein Mikroskop mit einem Beleuchtungsstrahlengang, bei dem durch Entfernen oder Lageverändern eines optischen Bauteiles am Objekt (21) eine Abdunkelung der Beleuchtung erzielt wird.

## Beschreibung

Die Erfindung betrifft ein Mikroskop mit einer Vorrichtung zur Reduktion der Beleuchtungsstärke der Mikroskopbeleuchtung an einer Pupille eines Patienten während der Betrachtung eines Auges durch das Mikroskop, wobei der Beleuchtungsstrahlengang auf das Patientenauge gerichtet bleibt, aber keine absolute Abdunkelung verursacht.

Zur Reduktion eines Gefahrenpotentials am Patientenauge wird in Operationspausen die Lichtstärke am zu operierenden Auge reduziert. In der Praxis erfolgt dies durch Abdecken des Auges mit der Hand oder durch eine Blende, die in den Beleuchtungsstrahlengang eingeschwenkt werden kann und auf die Augen-Pupille des Patienten abgebildet wird, so dass die Pupille durch die Blende abgeschattet wird. Solche Systeme sind u.A. auch als sogenannte "Eclipse-Filter" bekannt.

Solche Beleuchtungsvorrichtungen sind in grosser Zahl und verschieden Bauarten bekannt geworden; z.B. DE-U-9103433.7 und US-A-4715704.

Die DE-3339172-C2 beschreibt einen Voll-Blendenaufbau. Systeme mit Schwarzpunktenabdunkelungen wurden in der DE-AS-1951139 bekannt gemacht. Die DE-A-2654505 offenbart zwei ringförmige Blenden in einer Zwischenbildebene des Beleuchtungssystems, wobei die Bilder dieser Blenden in der Nähe der Iris auf der Augenlinse entstehen. Das DE-U-9301448 beschreibt eine vergleichbare Blendeneinrichtung mit einem teildurchlässigen Lichtfilter für den selben Zweck.

Alle bekannten Aufbauten gehen somit davon aus, dass man zur Abdunkelung etwas in den Strahlengang einschieben muss, damit das Patientenauge geschont wird.

Bei den bekannten Aufbauten sind um die entsprechenden Abbildeffekte der Blenden zu erzielen diese zusätzlich eingebrachten Elemente in der Nähe der Lichtquelle bzw. in der Nähe des Lichteintritts im System angeordnet. Dies führt dort zu konzentrierten Wärmeproblemen. Infolge der hohen Lichtleistung im Bereich der Lichtquelle oder des Lichteintritts (z.B. aus einem Lichtleiterkabel) kommt es zu einer starken Erwärmung der Blende. Dies kann bei genügend langer Einwirkdauer der Lichtleistung sogar zum Zerstören der Blende führen.

Abgesehen davon gibt die Blende die aus der Lichtstrahlung umgewandelte Wärme auch an den Mikroskopinnenraum ab, so dass es dort auch zu partieller Erwärmung kommen kann. Unliebsame Lichtreflexionen sind ebenfalls von Nachteil.

Ein weiterer Nachteil ergibt sich im Falle von Vollblenden, dass der Pupillenbereich völlig abgedunkelt ist und somit ein hoher Helligkeitsunterschied zwischen dem beleuchteten und abgedunkelten Zustand auftritt. Dies führt u.U. dazu, dass Details, die der Operateur auch in den Operationspausen im Bereich der Pupille erkennen möchte, nicht mehr ausreichend beleuchtet sind. Die letzterwähnte DE-U-9301448 schafft zwar hier Abhilfe, jedoch benötigt es dazu den relativ kostenaufwendigen Filter, der andererseits aber wieder die erwähnte Wärmeentwicklung mit sich bringt.

Der Erfindung liegt die Aufgabe zugrunde, ein neues verbessertes System zu schaffen, das einerseits die Erwärmung im üblichen Blendenbereich reduziert und andererseits zum Abdunkeln keine aufwendigen Filter benötigt.

Gelöst wird diese Aufgabe durch ein Mikroskop nach den Merkmalen des Anspruches 1 bzw. durch ein Verfahren nach den Merkmalen des Anspruches 5.

Durch das Entfernen eines optischen Bauelements (Linse) aus dem Strahlengang wird die beabsichtigte Funktion dieses Bauelements gelöscht. Da alle optischen Bauelemente im Strahlengang in der Regel der Bündelung bzw. Fokussierung des Lichtes dienen, führt das erfindungsgemässe Entfernen zu einem diffusen Streuen des Lichtes, was im Effekt zu einem deutlichen Abdunkeln der Lichtstärke im gefährdeten Bereich führt.

Unter Entfernung eines optischen Bauelementes ist im Sinne der Erfindung auch das Entfernen einer kompletten Baugruppe verschiedener Bauelemente zu verstehen oder auch das Verschieben oder Verschwenken der selben, so dass die Funktion der Fokussierung bzw. Bündelung des durch diese Baugruppe gestrahlten Lichtes nicht mehr gegeben ist.

Durch die verbleibende gestreute Beleuchtung wird aber immerhin noch so viel Licht durch den Beleuchtungsstrahlengang in Richtung Patientenauge geworfen, dass der Operateur oder das Hilfspersonal noch genügende Beleuchtung für die reine Beobachtung des Operationsortes vorfindet.

Die Erfindung ist nicht auf die Augenoperationsproblematik eingeschränkt, sondern kann überall dort eingesetzt werden, wo es um die Reduktion der Beleuchtungsstärke bei unveränderter Lichtleistung der Lichtquelle im Mikroskopiebereich geht. Die Konstanz der Farbtemperatur wird damit gewährleistet.

In den abhängigen Patentansprüchen sind weitere Verbesserungsmassnahmen beschrieben bzw. unter Schutz gestellt. Ein Ausführungsbeispiel der Erfindung ist in der Zeichnung bzw. Figurenbeschreibung angegeben.

Weitere Verbesserungen und erfindungsgemässe Details ergeben sich aus der Zeichnung und ihrer Beschreibung.

Die Figuren sind zusammenhängen beschrieben. Gleiche Bauteile tragen gleiche Bezugszeichen. Funktionsähnliche Bauteile tragen gleiche Bezugszeichen mit unterschiedlichen Indizes.

Es zeigen dabei:
- Fig. 1: zeigt einen Aufbau mit Lichteinführung über einen Lichtleiter und Kollektor- sowie Zoomlinsen und Prismen; eine der Kollektorlinsen ist mechanisch entfernbar; zusätzlich oder alternativ ist eine der Linsen am Prisma entfernbar;
- Fig. 2: zeigt einen vergleichbaren Aufbau zur Fig.1, wobei dort anstelle der Entfernung einer Zoomlinse das Prisma seitlich ausgeschwenkt bzw. herausgezogen werden kann.
- Fig. 3: zeigt einen Aufbau, bei dem die komplette Beleuchtungsoptik axial in Defokussierstellung verschiebbar ist.

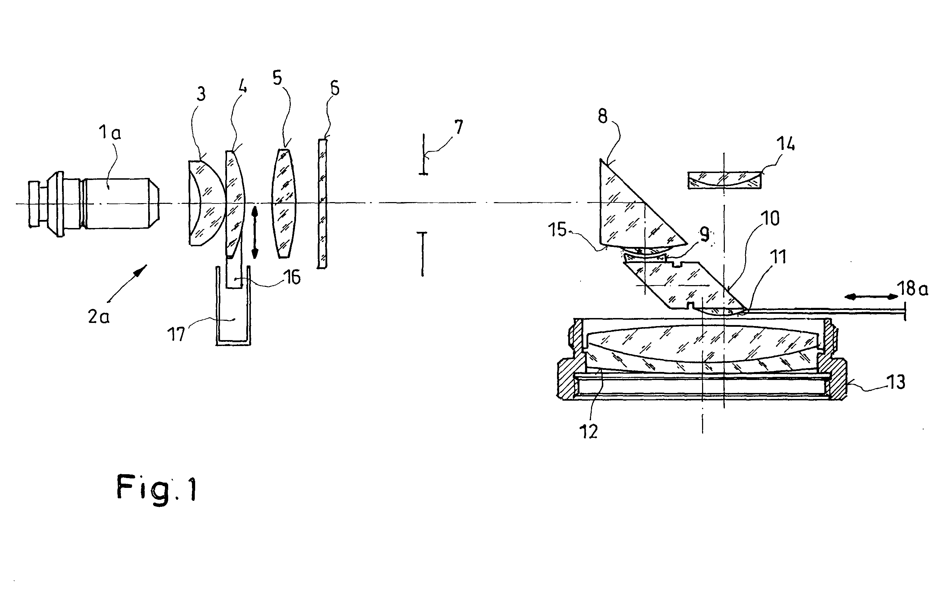

Fig.1 zeigt das Ende eines Lichtleiters 1a, dem eine Baugruppe optischer Elemente zur Beleuchtungsbündelung und Fokussierung 2a nachgeschaltet ist. Die Baugruppe 2a besteht beispielhaft aus einer Kollektorlinse 3, einer weiteren Kollektorlinse 4, einer Zoomlinse 5 und einem UV-Filter 6.

Im weiteren Beleuchtungsstrahlenverlauf folgt eine Blende 7, ein Spiegelprisma 8 mit einer konvexen Lichtaustrittsfläche 15, eine Konkavlinse 9, ein Spiegeltreppenprisma 10 mit einer Konvexlinse 11 und einem Hauptobjektiv 12, das in einer Objektivhalterung 13 gehalten ist.

Zusätzlich oder alternativ ist die Konvexlinse 11 entfernbar ausgebildet - ausschwenkbar oder herausziehbar gem. Pfeil 18a. Ist die Konvexlinse - wie u.U. bevorzugt - nicht entfernbar, kann sie auch am Prisma 10 angeklebt sein.

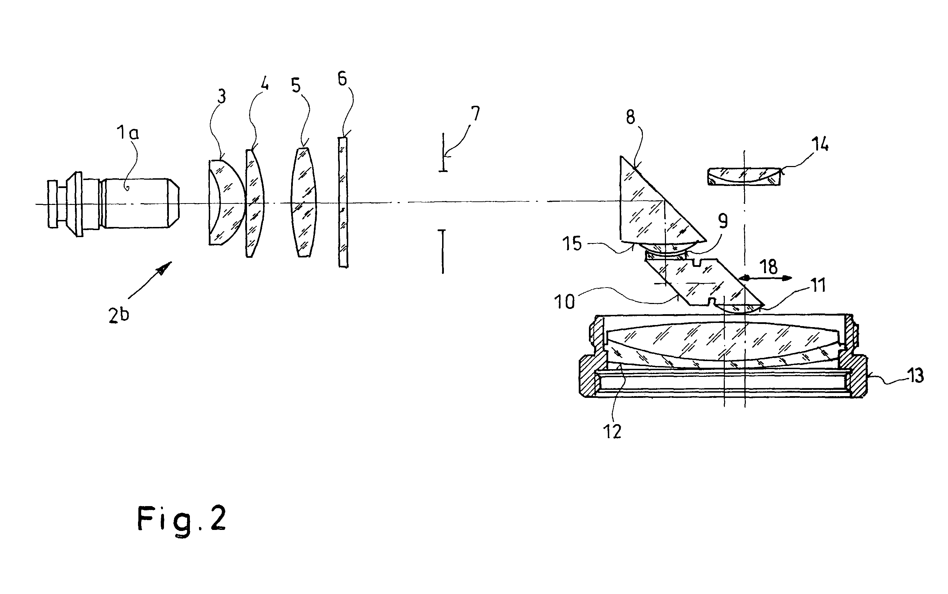

In Fig.1 und 2 ist auch eine Tubuslinsen 14 angedeutet, die dem Beobachtungsstrahlengang zugeordnet ist.

Das besondere und Neue an diesem Aufbau ist nun, dass die Kollektorlinse 4 aus der Baugruppe 2a mittels nur symbolisch dargestellter Mechanik entfernbar ist. Eine die Kollektorlinse 4 tragende Halterung 16 ist mit einem nicht dargestellten Griff oder motorischen Antrieb verbunden und kann in einer Führung verschoben werden, so dass sie aus der Baugruppe 2a fehlt und ihre Funktion entfällt.

Daraus ergibt sich der Effekt, dass aus der Baugruppe 2a nur mehr streuendes Licht austritt, dass im Zusammenwirken mit den Brechflächen an der konvexen Austrittsfläche 15 und den Linsen 9 und 11 in Verbindung mit dem Hauptobjektiv wieder nur ein defokussiertes, abgeschwächtes Licht auf das Objekt erlaubt.

Beim Aufbau nach Fig.2 bleibt die Baugruppe 2b statisch, jedoch wird zum Abdunkeln die Lichtkonzentrationsoptik mit den Linsen 9 und 11 seitlich herausgezogen - wie durch Pfeil 18 angedeutet - mittels nicht näher gezeigter mechanischer Angriffsmittel. Anstelle des Herausziehens ist auch ein Herausschwenken als Alternative denkbar. Das Prisma 10 kann dabei an seinem Platz verbleiben oder gegebenenfalls mit verschoben bzw. verschwenkt werden. Erfindungswichtig ist die Änderung der Lichtqualität in Richtung Beibehaltung einer gewissen Beleuchtung jedoch ohne Bündelung im kritischen Bereich des Patienten-Auges durch Entfernen eines lichtbündelnden Bauteils.

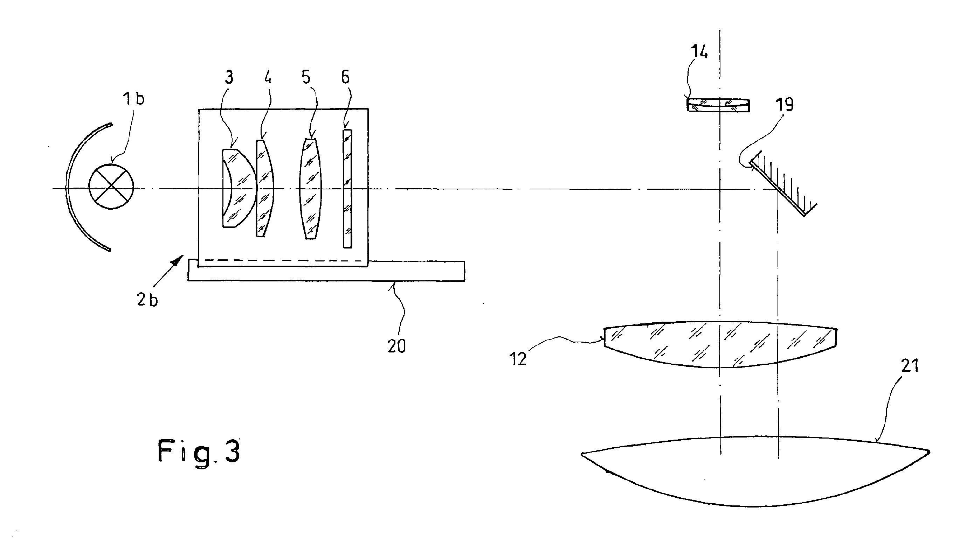

Bei der Variante nach Fig.3 wird anstelle des Entfernens eines Bauteils die Baugruppe 2d in einer Führung 20 entlang des Beleuchtungsstrahlengangs - mit nicht näher dargestellten Mitteln verschoben, um so im Abdunkelungsfall zu defokussieren bzw. zu streuen.

Im Rahmen der Erfindung sind noch verschiedenste Alternativen denkbar, die alle gemeinsam haben, dass nicht ein zusätzliches Element hinzugenommen wird, sondern ein bestehendes Element entfernt oder lageverändert wird, um dadurch den Lichtstrahlengang entsprechend zu verändern. So könnte z.B. der Spiegel 19 in Fig.3 geschwenkt oder so verschoben werden, dass das Licht nicht mehr fokussiert auf das Objekt 21 fällt.

### Bezugszeichenliste

- 1a: Lichtleiter
- 1b: Lampe
- 2a,b: Baugruppe optischer Elemente
- 3: erste Kollektorlinse
- 4: zweite Kollektorlinse
- 5: Zoomlinse
- 6: UV-Filter
- 7: Blende
- 8: Spiegelprisma
- 9: Konkav-Linse
- 10: Spiegeltreppenprisma
- 11: Konvex-Linse
- 12: Hauptobjektiv
- 13: Objektivhalterung
- 14: Tubuslinse
- 15: konvexe Austrittsfläche
- 16: Halterung
- 17: Führung
- 18, 18a: Pfeil
- 19: Spiegel

## Patentansprüche

1. Mikroskop, insbesondere Operationsmikroskop mit einer Beleuchtungseinrichtung für das Objekt (21), die einen Lichtstrahlengang durch das Hauptobjektiv (12) oder im Bereich des Hauptobjektivs mit optischen Bauelementen (2-12) für den Lichtstrahlengang und eine Vorrichtung für das Abdunkeln der Beleuchtungsstärke am Objekt (21) aufweist, **dadurch gekennzeichnet, dass** die Vorrichtung einen Mechanismus zum Entfernen oder Lageverändern wenigstens eines der optischen Bauelemente (2-12) umfasst, so dass im Betriebsfall durch das Entfernen oder Lageverändern eine Abdunkelung am Objekt eintritt.

2. Mikroskop nach Anspruch 1 mit wenigstens einer Kollektorlinse (4) im Lichtstrahlengang, **dadurch gekennzeichnet, dass** die Kollektorlinse (4) mittels händisch oder elektromechanisch bedienbarem Antrieb aus dem Lichtstrahlengang entfernbar ist.

3. Mikroskop nach Anspruch 1 oder 2 mit einem Spiegel(treppen)prisma (10) mit einer Lichtkonzentrationsoptik (9,11) zur Weiterleitung des Lichtstrahls durch das Hauptobjektiv (12), **dadurch gekennzeichnet, dass** die Lichtkonzentrationsoptik (9,11) oder wenigstens ein Teil davon aus dem Lichtstrahlengang entfernbar - vorzugsweise herausziebar oder herausschwenkbar - ist.

4. Mikroskop nach Anspruch 1, 2 oder 3, mit einer Baugruppe (2) optischer Elemente im Lichtstrahlengang, **dadurch gekennzeichnet, dass** die Baugruppe (2b) aus dem Lichtstrahlengang entfernbar oder entlang des Lichtstrahlenganges verschiebbar ist.

5. Verfahren zum Abdunkeln eines beleuchteten Objektes (21) unter einem Mikroskop, insbesondere unter einem Operationsmikroskop mit einer Beleuchtungseinrichtung mit einem integrierten Beleuchtungsstrahlengang mit optischen Bauelementen (2-12), **dadurch gekennzeichnet, dass** wenigstens eines der Bauelemente (2-12) oder eine Gruppe (2) dieser Bauelemente (2-12) aus dem Beleuchtungsstrahlengang entfernt oder in diesem so verschoben wird, dass das Licht am Objekt gestreut bzw. defokussiert auftrifft.

6. Verfahren nach Anspruch 5, **dadurch gekennzeichnet, dass** eine Kollektorlinse (4) entfernt wird.

7. Verfahren nach Anspruch 5 oder 6, **dadurch gekennzeichnet, dass** Elemente zur Lichtkonzentration (9,11) entfernt werden.

8. Verfahren nach einem der Ansprüche 5 bis 7, **dadurch gekennzeichnet, dass** ein Spiegel (19) verschwenkt wird.

9. Verfahren nach einem der vorhergehenden Ansprüche 5 bis 8, **dadurch gekennzeichnet, dass** wenigstens eine Baugruppe (2b) von optischen Bauelementen (2-12) entlang des Lichtstrahlengangs verschoben wird.
